# EUROPEAN PATENT SPECIFICATION

(11) **EP 4 560 369 B1**
(45) Date of publication and mention of the grant of the patent: **02.12.2026**
(21) Application number: 25169863.5
(22) Date of filing: 08.08.2022
(51) Int. Cl.: A61B 18/24, G02B 6/38

(54) **CONNECTOR FOR MULTIPLEXER OPTICAL COUPLING**
STECKER FÜR OPTISCHE MULTIPLEXERKOPPLUNG
CONNECTEUR POUR COUPLAGE OPTIQUE DE MULTIPLEXEUR

(30) Priority: 24.08.2021 US 202163236633 P; 07.08.2022 US 202217882586
(43) Date of publication of application: 28.05.2025
(62) Document number(s) of the earlier application(s) in accordance with Art. 76 EPC: 22764507.4 / 4 391 946
(73) Proprietor: Boston Scientific Scimed, Inc., Maple Grove, Minnesota 55311 (US)
(72) Inventor: Cook, Christopher A., Laguna Niguel, 92677 (US); Schultheis, Eric, San Clemente, 92673 (US)
(74) Representative: Vossius & Partner Patentanwälte Rechtsanwälte mbB

(56) References cited:
- US-A1- 2011 184 244
- US-A1- 2014 114 198
- US-A1- 2016 018 602

## Description

### BACKGROUND

Vascular lesions within vessels in the body can be associated with an increased risk for major adverse events, such as myocardial infarction, embolism, deep vein thrombosis, stroke, and the like. Severe vascular lesions can be difficult to treat and achieve patency for a physician in a clinical setting.

Vascular lesions may be treated using interventions such as drug therapy, balloon angioplasty, atherectomy, stent placement, vascular graft bypass, to name a few. Such interventions may not always be ideal or may require subsequent treatment to address the lesion. In this context, technical teaching provided in US2016/0018602 A1 is acknowledged.

### SUMMARY

The present invention is defined by the appended set of claims.

The present invention is directed toward a catheter system for treating a treatment site within or adjacent to a vessel wall or a heart valve. The
catheter system includes a light source, a first light guide, a second light guide, and a guide bundle. The light source generates light energy. The first light guide receives the light energy from the light source. The first light guide has a guide proximal end. The second light guide receives the light energy from the light source. The second light guide has a guide proximal end. A guide bundle is in optical communication with the light source. The guide bundle bundles the first light guide and the second light guide. The guide bundle includes a first ferrule that engages the guide proximal end of the first light guide, and a second ferrule that engages the guide proximal end of the second light guide.

The guide bundle further includes (i) a first ferrule assembly including the first ferrule, and (ii) a second ferrule assembly including the second ferrule.

In certain embodiments, at least one of the first ferrule and the second ferrule are formed at least partially from a ceramic material.

In various embodiments, at least one of the first ferrule and the second ferrule are formed at least partially from a metallic material.

In some embodiments, the first ferrule assembly further includes a first spring that engages the first ferrule and the second ferrule assembly further includes a second spring that engages the second ferrule.

The catheter system further includes a receptacle assembly that receives the first ferrule and the second ferrule.

In various embodiments, the receptacle assembly is formed at least partially from a ceramic material.

In some embodiments, the receptacle assembly is formed at least partially from a metallic material.

In certain embodiments, the receptacle assembly includes at least one alignment guide that is configured to guide the alignment of the receptacle assembly and the guide bundle.

In various embodiments, the alignment guide is a guide pin.

In some embodiments, the alignment guide is a guide tongue of a tongue and groove system.

In certain embodiments, the alignment guide is a guide rail.

The receptacle assembly includes a receptacle block that is coupled to a backing plate, the backing plate being configured to engage the first ferrule and the second ferrule.

In some embodiments, each of the ferrules includes a proximal end face, wherein the backing plate is configured to engage each of the proximal end faces.

In certain embodiments, the backing plate includes (i) a first alignment hole that is configured to align a first guide beam with the first light guide, and (ii) a second alignment hole that is configured to align a second guide beam with the second light guide.

The receptacle assembly includes (i) a first receptacle hole that is configured to receive the first ferrule, and (ii) a second receptacle hole that is configured to receive the second ferrule.

In some embodiments, the first receptacle hole and the second receptacle hole are each formed in the receptacle block, the first receptacle hole and the second receptacle hole being aligned on a same linear axis as one another.

According to the present invention, the first receptacle hole and the second receptacle hole are each formed in the receptacle block as v-grooves.

In various embodiments, the receptacle assembly includes a retainer assembly that retains (i) the first ferrule in the first receptacle hole, and (ii) the second ferrule in the second receptacle hole.

In some embodiments, the retainer assembly includes a clamping bar having a ball spring plunger, the ball spring plunger being configured to contact (i) at least a portion of the first ferrule so that the first ferrule is retained in the first receptacle hole, and (ii) at least a portion of the second ferrule so that the second ferrule is retained in the second receptacle hole.

In certain embodiments, the clamping bar is configured to be rotatable about a clamping bar axis.

In various embodiments, the clamping bar includes a compliant material that is configured to spread a retaining force across the ferrules so that each of the ferrules is retained within a corresponding receptacle hole.

The present disclosure is also directed toward a method for treating a vascular lesion within or adjacent to a vessel wall using the catheter system of any of the embodiments described herein.

The present disclosure is further directed toward a method for manufacturing the catheter system of any of the embodiments described herein.

The present disclosure is also directed toward a catheter system for treating a treatment site within or adjacent to a vessel wall or a heart valve. In various embodiments, the catheter system includes a light source, a plurality of light guides, a guide bundle, and a receptacle assembly. The light source generates light energy. The plurality of light guides each individually receives the light energy from the light source. Each of the plurality of light guides has a corresponding guide proximal end. The guide bundle is in optical communication with the light source. The guide bundle bundles the plurality of light guides. The guide bundle includes a plurality of ferrules that each engages one of the guide proximal ends of a corresponding light guide. The receptacle assembly receives and aligns the plurality of ferrules into one of (i) a circular pattern, and (ii) a hexagonal pattern.

In some embodiments, at least one of the light guides is an optical fiber.

In certain embodiments, the light source is a laser.

This summary is an overview of some of the teachings of the present application and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Figure 1 is a simplified, cross-sectional view of an embodiment of a catheter system in accordance with various embodiments herein, the catheter system including a multiplexer and a guide bundle having features of the present invention;
Figure 2 is a top view of a portion of one embodiment of the catheter system including an embodiment of a guide bundle;
Figure 3 is a perspective front view of a portion of an embodiment of the catheter system including an embodiment of the guide bundle;
Figure 4 is a perspective front view of a portion of an embodiment of the catheter system including one embodiment of a receptacle assembly;
Figure 5 is a perspective rear view of a portion of an embodiment of the catheter system including one embodiment of the receptacle assembly;
Figure 6 is a top view of a portion of an embodiment of the catheter system including an embodiment of the guide bundle and the receptacle assembly, the guide bundle being shown in a partially connected state with respect to the receptacle assembly;
Figure 7 is a simplified cross-sectional view of a portion of an embodiment of the catheter system taken on line 7-7 in Figure 6, the catheter system including one embodiment of a guide coupling housing and the receptacle assembly, the guide coupling housing being shown in a partially connected state with respect to the receptacle assembly;
Figure 8 is a perspective front view of a portion of an embodiment of the catheter system including one embodiment of the receptacle assembly;
Figure 9 is a front elevation view of a portion of an embodiment of the catheter system including one embodiment of a portion of the receptacle assembly; and
Figure 10 is a simplified cross-sectional view of a portion of an embodiment of the catheter system taken on line 10-10 in Figure 9, the catheter system including one embodiment of the receptacle assembly.

While embodiments of the present invention are susceptible to various modifications and alternative forms, specifics thereof have been shown by way of example and drawings, and are described in detail herein. It is understood, however, that the scope herein is not limited to the particular embodiments described.

### DESCRIPTION

Treatment of vascular lesions (also sometimes referred to herein as "treatment sites") can reduce major adverse events or death in affected subjects. As referred to herein, a major adverse event is one that can occur anywhere within the body due to the presence of a vascular lesion. Major adverse events can include but are not limited to, major adverse cardiac events, major adverse events in the peripheral or central vasculature, major adverse events in the brain, major adverse events in the musculature, or major adverse events in any of the internal organs.

As used herein, the terms "intravascular lesion", "vascular lesion" and "treatment site" are used interchangeably unless otherwise noted. The intravascular lesions and/or the vascular lesions are sometimes referred to herein simply as "lesions". Also, as used herein, the terms "focused location" and "focused spot" can be used interchangeably unless otherwise noted and can refer to any location where the light energy is focused to a small diameter than the initial diameter of the light source.

Those of ordinary skill in the art will realize that the following detailed description of the present invention is illustrative only and is not intended to be in any way limiting. Other embodiments of the present invention will readily suggest themselves to such skilled persons having the benefit of this disclosure. Reference will now be made in detail to implementations of the present invention as illustrated in the accompanying drawings.

In the interest of clarity, not all of the routine features of the implementations described herein are shown and described. It will, of course, be appreciated that in the development of any such actual implementation, numerous implementation-specific decisions must be made in order to achieve the developer's specific goals, such as compliance with application-related and business-related constraints, and that these specific goals will vary from one implementation to another and from one developer to another. Moreover, it is appreciated that such a development effort might be complex and time-consuming, but would nevertheless be a routine undertaking of engineering for those of ordinary skill in the art having the benefit of this disclosure.

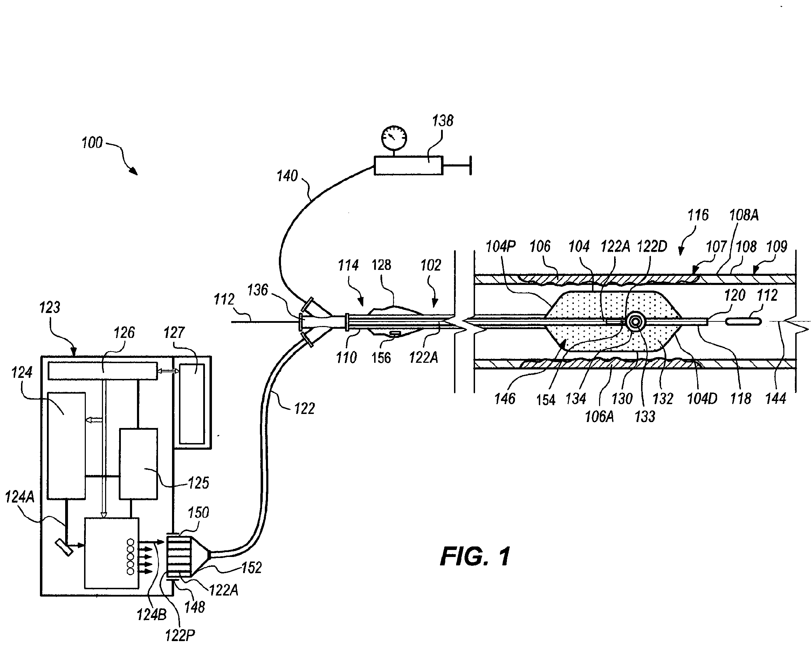

The catheter systems disclosed herein can include many different forms. Referring now to Figure 1, a schematic cross-sectional view is shown of a catheter system 100 in accordance with various embodiments. The catheter system 100 is suitable for imparting pressure waves to induce fractures in one or more treatment sites within or adjacent to a vessel wall of a blood vessel, or on or adjacent to a heart valve, within a body of a patient. In the embodiment illustrated in Figure 1, the catheter system 100 can include one or more of a catheter 102, an energy guide bundle 122 including one or more energy guides 122A (some embodiments described here include at least a first energy guide and a second energy guide), a source manifold 136, a fluid pump 138, a multiplexer 123 including one or more of an energy source 124, a power source 125, a system controller 126, and a graphic user interface 127 (a "GUI"), and a handle assembly 128. Alternatively, the catheter system 100 can include more components or fewer components than those specifically illustrated and described in relation to Figure 1.

It is appreciated that in some embodiments the energy guide bundle 122 shown and described can be a light guide bundle 122, which can include one or more light guides 122A. In certain embodiments, the energy source 124 can include a light source 124. For example, the energy guides 122A can be optical fibers, and/or the energy source 124 can include a laser. Alternatively, the energy guide bundle 122 can include different types of energy guides 122A (such as electrodes or electrode pairs), and/or a different type of energy source 124 (such as a high voltage energy source, for example). It is understood that the energy guides 122A and/or the energy source 124 can include any suitable type of energy guides or energy sources that can generate and/or transmit energy.

In various embodiments, the catheter 102 is configured to move to a treatment site 106 within or adjacent to a vessel wall 108A of a blood vessel 108 within a body 107 of a patient 109. The treatment site 106 can include one or more vascular lesions 106A such as calcified vascular lesions, for example. Additionally, or in the alternative, the treatment site 106 can include vascular lesions 106A such as fibrous vascular lesions. Still alternatively, in some implementations, the catheter 102 can be used at a treatment site 106 within or adjacent to a heart valve within the body 107 of the patient 109.

The catheter 102 can include an inflatable balloon 104 (sometimes referred to herein simply as a "balloon"), a catheter shaft 110, and a guidewire 112. The balloon 104 can be coupled to the catheter shaft 110. The balloon 104 can include a balloon proximal end 104P and a balloon distal end 104D. The catheter shaft 110 can extend from a proximal portion 114 of the catheter system 100 to a distal portion 116 of the catheter system 100. The catheter shaft 110 can include a longitudinal axis 144. The catheter shaft 110 can also include a guidewire lumen 118 which is configured to move over the guidewire 112. As utilized herein, the guidewire lumen 118 defines a conduit through which the guide wire 112 extends. The catheter shaft 110 can further include an inflation lumen (not shown) and/or various other lumens for various other purposes. In some embodiments, the catheter 102 can have a distal end opening 120 and can accommodate and be tracked over the guidewire 112 as the catheter 102 is moved and positioned at or near the treatment site 106. In some embodiments, the balloon proximal end 104P can be coupled to the catheter shaft 110, and the balloon distal end 104D can be coupled to the guidewire lumen 118.

The balloon 104 includes a balloon wall 130 that defines a balloon interior 146. The balloon 104 can be selectively inflated with a balloon fluid 132 to expand from a deflated state suitable for advancing the catheter 102 through a patient's vasculature, to an inflated state (as shown in Figure 1) suitable for anchoring the catheter 102 in position relative to the treatment site 106. Stated in another manner, when the balloon 104 is in the inflated state, the balloon wall 130 of the balloon 104 is configured to be positioned substantially adjacent to the treatment sites 106. It is appreciated that although Figure 1 illustrates the balloon wall 130 of the balloon 104 is shown spaced apart from the treatment site 106 of the blood vessel 108 when in the inflated state, this is done merely for ease of illustration. It is recognized that the balloon wall 130 of the balloon 104 will typically be substantially directly adjacent to and/or abutting the treatment site 106 when the balloon 104 is in the inflated state.

The balloon 104 suitable for use in the catheter system 100 includes those that can be passed through the vasculature of a patient 109 when in the deflated state. In some embodiments, the balloon 104 is made from silicone. In other embodiments, the balloon 104 can be made from polydimethylsiloxane (PDMS), polyurethane, polymers such as PEBAX^{™} material, nylon, or any other suitable material.

The balloon 104 can have any suitable diameter (in the inflated state). In various embodiments, the balloon 104 can have a diameter (in the inflated state) ranging from less than one millimeter (mm) up to 25 mm. In some embodiments, the balloon 104 can have a diameter (in the inflated state) ranging from at least 1.5 mm up to 14 mm. In some embodiments, the balloons104 can have a diameter (in the inflated state) ranging from at least two mm up to five mm.

In some embodiments, the balloon 104 can have a length ranging from at least three mm to 300 mm. More particularly, in some embodiments, the balloon 104 can have a length ranging from at least eight mm to 200 mm. It is appreciated that a balloon 104 having a relatively longer length can be positioned adjacent to larger treatment sites 106, and, thus, may be used for imparting pressure waves onto and inducing fractures in larger vascular lesions 106A or multiple vascular lesions 106A at precise locations within the treatment site 106. It is further appreciated that a longer balloon 104 can also be positioned adjacent to multiple treatment sites 106 at any one given time.

The balloon 104 can be inflated to inflation pressures of between approximately one atmosphere (atm) and 70 atm. In some embodiments, the balloon 104 can be inflated to inflation pressures of from at least 20 atm to 60 atm. In other embodiments, the balloon 104 can be inflated to inflation pressures of from at least six atm to 20 atm. In still other embodiments, the balloon 104 can be inflated to inflation pressures of from at least three atm to 20 atm. In yet other embodiments, the balloon 104 can be inflated to inflation pressures of from at least two atm to ten atm.

The balloon 104 can have various shapes, including, but not to be limited to, a conical shape, a square shape, a rectangular shape, a spherical shape, a conical/square shape, a conical/spherical shape, an extended spherical shape, an oval shape, a tapered shape, a bone shape, a stepped diameter shape, an offset shape, or a conical offset shape. In some embodiments, the balloon 104 can include a drug eluting coating or a drug eluting stent structure. The drug eluting coating or drug eluting stent can include one or more therapeutic agents including anti-inflammatory agents, anti-neoplastic agents, anti-angiogenic agents, and the like.

The balloon fluid 132 can be a liquid or a gas. Some examples of the balloon fluid 132 suitable for use can include, but are not limited to one or more of water, saline, contrast medium, fluorocarbons, perfluorocarbons, gases, such as carbon dioxide, or any other suitable balloon fluid 132. In some embodiments, the balloon fluid 132 can be used as a base inflation fluid. In some embodiments, the balloon fluid 132 can include a mixture of saline to contrast medium in a volume ratio of approximately 50:50. In other embodiments, the balloon fluid 132 can include a mixture of saline to contrast medium in a volume ratio of approximately 25:75. In still other embodiments, the balloon fluid 132 can include a mixture of saline to contrast medium in a volume ratio of approximately 75:25. However, it is understood that any suitable ratio of saline to contrast medium can be used. The balloon fluid 132 can be tailored on the basis of composition, viscosity, and the like so that the rate of travel of the pressure waves are appropriately manipulated. In certain embodiments, the balloon fluid 132 suitable for use herein is biocompatible. A volume of balloon fluid 132 can be tailored by the chosen light source 124 and the type of balloon fluid 132 used.

In some embodiments, the contrast agents used in the contrast media can include, but are not to be limited to, iodine-based contrast agents, such as ionic or non-ionic iodine-based contrast agents. Some non-limiting examples of ionic iodine-based contrast agents include diatrizoate, metrizoate, iothalamate, and ioxaglate. Some non-limiting examples of non-ionic iodine-based contrast agents include iopamidol, iohexol, ioxilan, iopromide, iodixanol, and ioversol. In other embodiments, non-iodine based contrast agents can be used. Suitable non-iodine containing contrast agents can include gadolinium (III)-based contrast agents. Suitable fluorocarbon and perfluorocarbon agents can include, but are not to be limited to, agents such as perfluorocarbon dodecafluoropentane (DDFP, C5F12).

The balloon fluids 132 can include those that include absorptive agents that can selectively absorb light in the ultraviolet region (e.g., at least ten nanometers (nm) to 400 nm), the visible region (e.g., at least 400 nm to 780 nm), or the near-infrared region (e.g., at least 780 nm to 2.5 µm) of the electromagnetic spectrum. Suitable absorptive agents can include those with absorption maxima along the spectrum from at least ten nm to 2.5 µm. Alternatively, the balloon fluid 132 can include absorptive agents that can selectively absorb light in the mid-infrared region (e.g., at least 2.5 µm to 15 µm), or the far-infrared region (e.g., at least 15 µm to one mm) of the electromagnetic spectrum. In various embodiments, the absorptive agent can be those that have an absorption maximum matched with the emission maximum of the laser used in the catheter system 100. By way of non-limiting examples, various lasers described herein can include neodymium:yttrium-aluminum-garnet (Nd:YAG - emission maximum = 1064 nm) lasers, holmium:YAG (Ho:YAG - emission maximum = 2.1 µm) lasers, or erbium:YAG (Er:YAG - emission maximum = 2.94 µm) lasers. In some embodiments, the absorptive agents can be water soluble. In other embodiments, the absorptive agents are not water soluble. In some embodiments, the absorptive agents used in the balloon fluids 132 can be tailored to match the peak emission of the light source 124. Various light sources 124 having emission wavelengths of at least ten nanometers to one millimeter are discussed elsewhere herein.

The catheter shaft 110 of the catheter 102 can be coupled to the one or more light guides 122A of the light guide bundle 122 that are in optical communication with the light source 124. The light guide(s) 122A can be disposed along the catheter shaft 110 and within the balloon 104. Each of the light guides 122A can have a guide distal end 122D that is at any suitable longitudinal position relative to a length of the balloon 104. In some embodiments, each light guide 122A can be an optical fiber, and the light source 124 can be a laser. The light source 124 can be in optical communication with the light guides 122A at the proximal portion 114 of the catheter system 100. More particularly, the light source 124 can selectively, simultaneously, sequentially, and/or be in optical communication with each of the light guides 122A in any desired combination, order, and/or pattern due to the presence and operation of the multiplexer 128.

In some embodiments, the catheter shaft 110 can be coupled to multiple light guides 122A such as a first light guide, a second light guide, a third light guide, etc., which can be disposed at any suitable positions about the guidewire lumen 118 and/or the catheter shaft 110. For example, in certain non-exclusive embodiments, two light guides 122A can be spaced apart by approximately 180 degrees about the circumference of the guidewire lumen 118 and/or the catheter shaft 110; three light guides 122A can be spaced apart by approximately 120 degrees about the circumference of the guidewire lumen 118 and/or the catheter shaft 110, or four light guides 122A can be spaced apart by approximately 90 degrees about the circumference of the guidewire lumen 118 and/or the catheter shaft 110. Still alternatively, multiple light guides 122A need not be uniformly spaced apart from one another about the circumference of the guidewire lumen 118 and/or the catheter shaft 110. More particularly, the light guides 122A can be disposed either uniformly or non-uniformly about the guidewire lumen 118 and/or the catheter shaft 110 to achieve the desired effect in the desired locations.

The catheter system 100 and/or the light guide bundle 122 can include any number of light guides 122A in optical communication with the light source 124 at the proximal portion 114, and with the balloon fluid 132 within the balloon interior 146 of the balloon 104 at the distal portion 116. For example, in some embodiments, the catheter system 100 and/or the light guide bundle 122 can include from one light guide 122A to five light guides 122A. In other embodiments, the catheter system 100 and/or the light guide bundle 122 can include from five light guides 122A to fifteen light guides 122A. In yet other embodiments, the catheter system 100 and/or the light guide bundle 122 can include from ten light guides 122A to thirty light guides 122A. Alternatively, in still other embodiments, the catheter system 100 and/or the light guide bundle 122 can include greater than 30 light guides 122A.

The light guides 122A can have any suitable design for purposes of generating plasma and/or pressure waves in the balloon fluid 132 within the balloon interior 146. In certain embodiments, the light guides 122A can include an optical fiber or flexible light pipe. The light guides 122A can be thin and flexible and can allow light signals to be sent with very little loss of strength. The light guides 122A can include a core surrounded by a cladding about its circumference. In some embodiments, the core can be a cylindrical core or a partially cylindrical core. The core and cladding of the light guides 122A can be formed from one or more materials, including but not limited to one or more types of glass, silica, or one or more polymers. The light guides 122A may also include a protective coating, such as a polymer. It is appreciated that the index of refraction of the core will be greater than the index of refraction of the cladding.

Each light guide 122A can guide light energy along its length from a guide proximal end 122P to the guide distal end 122D having at least one optical window (not shown) that is positioned within the balloon interior 146.

The light guides 122A can assume many configurations about and/or relative to the catheter shaft 110 of the catheter 102. In some embodiments, the light guides 122A can run parallel to the longitudinal axis 144 of the catheter shaft 110. In some embodiments, the light guides 122A can be physically coupled to the catheter shaft 110. In other embodiments, the light guides 122A can be disposed along a length of an outer diameter of the catheter shaft 110. In yet other embodiments, the light guides 122A can be disposed within one or more light guide lumens within the catheter shaft 110.

The light guides 122A can also be disposed at any suitable positions about the circumference of the guidewire lumen 118 and/or the catheter shaft 110, and the guide distal end 122D of each of the light guides 122A can be disposed at any suitable longitudinal position relative to the length of the balloon 104 and/or relative to the length of the guidewire lumen 118 to more effectively and precisely impart pressure waves for purposes of disrupting the vascular lesions 106A at the treatment site 106.

In certain embodiments, the light guides 122A can include one or more photoacoustic transducers 154, where each photoacoustic transducer 154 can be in optical communication with the light guide 122A within which it is disposed. In some embodiments, the photoacoustic transducers 154 can be in optical communication with the guide distal end 122D of the light guide 122A. Additionally, in such embodiments, the photoacoustic transducers 154 can have a shape that corresponds with and/or conforms to the guide distal end 122D of the light guide 122A.

The photoacoustic transducer 154 is configured to convert light energy into an acoustic wave at or near the guide distal end 122D of the light guide 122A. The direction of the acoustic wave can be tailored by changing an angle of the guide distal end 122D of the light guide 122A.

In certain embodiments, the photoacoustic transducers 154 disposed at the guide distal end 122D of the light guide 122A can assume the same shape as the guide distal end 122D of the light guide 122A. For example, in certain non-exclusive embodiments, the photoacoustic transducer 154 and/or the guide distal end 122D can have a conical shape, a convex shape, a concave shape, a bulbous shape, a square shape, a stepped shape, a half-circle shape, an ovoid shape, and the like. The light guide 122A can further include additional photoacoustic transducers 154 disposed along one or more side surfaces of the length of the light guide 122A.

In some embodiments, the light guides 122A can further include one or more diverting features or "diverters" (not shown in Figure 1) within the light guide 122A that are configured to direct light to exit the light guide 122A toward a side surface which can be located at or near the guide distal end 122D of the light guide 122A, and toward the balloon wall 130. A diverting feature can include any feature of the system that diverts light energy from the light guide 122A away from its axial path toward a side surface of the light guide 122A. Additionally, the light guides 122A can each include one or more light windows disposed along the longitudinal or circumferential surfaces of each light guide 122A and in optical communication with a diverting feature. Stated in another manner, the diverting features can be configured to direct light energy in the light guide 122A toward a side surface that is at or near the guide distal end 122D, where the side surface is in optical communication with a light window. The light windows can include a portion of the light guide 122A that allows light energy to exit the light guide 122A from within the light guide 122A, such as a portion of the light guide 122A lacking a cladding material on or about the light guide 122A.

Examples of the diverting features suitable for use include a reflecting element, a refracting element, and a fiber diffuser. The diverting features suitable for focusing light energy away from the tip of the light guides 122A can include but are not to be limited to, those having a convex surface, a gradient-index (GRIN) lens, and a mirror focus lens. Upon contact with the diverting feature, the light energy is diverted within the light guide 122A to one or more of a plasma generator 133 and the photoacoustic transducer 154 that is in optical communication with a side surface of the light guide 122A. As noted, the photoacoustic transducer 154 then converts light energy into an acoustic wave that extends away from the side surface of the light guide 122A.

The source manifold 136 can be positioned at or near the proximal portion 114 of the catheter system 100. The source manifold 136 can include one or more proximal end openings that can receive the one or more light guides 122A of the light guide bundle 122, the guidewire 112, and/or an inflation conduit 140 that is coupled in fluid communication with the fluid pump 138. The catheter system 100 can also include the fluid pump 138 that is configured to inflate the balloon 104 with the balloon fluid 132 as needed.

As noted above, in the embodiment illustrated in Figure 1, the multiplexer 123 includes one or more of the light source 124, the power source 125, the system controller 126, and the GUI 127. Alternatively, the multiplexer 123 can include more components or fewer components than those specifically illustrated in Figure 1. For example, in certain non-exclusive alternative embodiments, the multiplexer 123 can be designed without the GUI 127. Still alternatively, one or more of the light source 124, the power source 125, the system controller 126, and the GUI 127 can be provided within the catheter system 100 without the specific need for the multiplexer 123.

In some embodiments, the multiplexer 123 can include a two-channel splitter design. The light guide bundle 122 can include a manual positioning mechanism that is mounted on an optical breadboard and/or platen. This design enables linear positional adjustment and array tilting by rotating about a channel one light guide 122A axis (not shown in Figure 1). The adjustment method, in other embodiments, can include at least two adjustment steps, 1) aligning the planar positions of the source beam 124B at Channel 1, and 2) adjusting the light guide bundle 122 to achieve the best alignment at Channel 10.

As shown, the multiplexer 123, and the components included therewith, is operatively coupled to the catheter 102, the light guide bundle 122, and the remainder of the catheter system 100. For example, in some embodiments, as illustrated in Figure 1, the multiplexer 123 can include a console connection aperture 148 (also sometimes referred to generally as a "socket") by which the light guide bundle 122 is mechanically coupled to the multiplexer 123. In such embodiments, the light guide bundle 122 can include a guide coupling housing 150 that houses a portion, e.g., the guide proximal end 122P, of each of the light guides 122A. The guide coupling housing 150 is configured to fit and be selectively retained within the console connection aperture 148 to provide the mechanical coupling between the light guide bundle 122 and the multiplexer 123.

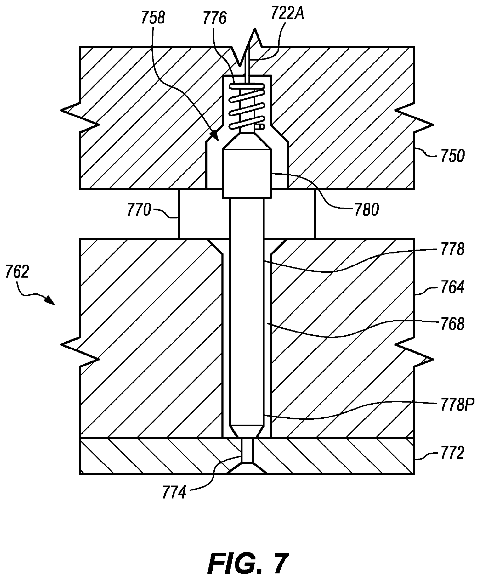

The light guide bundle 122 can also include a guide bundle 152 (or "shell") that brings each of the individual light guides 122A closer together so that the light guides 122A and/or the light guide bundle 122 can be in a more compact form as it extends with the catheter 102 into the blood vessel 108 during use of the catheter system 100. In some embodiments, the light guides 122A leading to the plasma generator 133 can be organized into a light guide bundle 122 including a linear block with an array of precision holes forming a multi-channel ferrule 778 (illustrated in Figure 7, for example). In other embodiments, the light guide bundle 122 could include a mechanical connector array or block connector that organizes singular ferrules 778 into one of (i) a linear array, (ii) a circular pattern, and (iii) a hexagonal pattern.

The light source 124 can be selectively and/or alternatively coupled in optical communication with each of the light guides 122A, i.e. to the guide proximal end 122P of each of the light guides 122A, in the light guide bundle 122. In particular, the light source 124 is configured to generate light energy in the form of a source beam 124A, such as a pulsed source beam, that can be selectively and/or alternatively directed to and received by each of the light guides 122A in the light guide bundle 122 as an individual guide beam 124B. Alternatively, the catheter system 100 can include more than one light source 124. For example, in one non-exclusive alternative embodiment, the catheter system 100 can include a separate light source 124 for each of the light guides 122A in the light guide bundle 122. The light source 124 can be operated at low energies.

The light source 124 can have any suitable design. In certain embodiments, the light source 124 can be configured to provide sub-millisecond pulses of light energy from the light source 124 that are focused onto a small spot in order to couple it into the guide proximal end 122P of the light guide 122A. Such pulses of light energy are then directed and/or guided along the light guides 122A to a location within the balloon interior 146 of the balloon 104, thereby inducing plasma formation (also sometimes referred to herein as a "plasma flash") in the balloon fluid 132 within the balloon interior 146 of the balloon 104, such as via the plasma generator 133 that can be located at the guide distal end 122D of the light guide 122A. In particular, the light emitted at the guide distal end 122D of the light guide 122A energizes the plasma generator 133 to form the plasma within the balloon fluid 132 within the balloon interior 146. The plasma formation causes rapid bubble formation and imparts pressure waves upon the treatment site 106. An exemplary plasma-induced bubble 134 is illustrated in Figure 1.

In various non-exclusive alternative embodiments, the sub-millisecond pulses of light energy from the light source 124 can be delivered to the treatment site 106 at a frequency of between approximately one hertz (Hz) and 5000 Hz, between approximately 30 Hz and 1000 Hz, between approximately ten Hz and 100 Hz, or between approximately one Hz and 30 Hz. Alternatively, the sub-millisecond pulses of light energy can be delivered to the treatment site 106 at a frequency that can be greater than 5000 Hz or less than one Hz, or any other suitable range of frequencies.

It is appreciated that although the light source 124 is typically utilized to provide pulses of light energy, the light source 124 can still be described as providing a single source beam 124A, i.e. a single pulsed source beam.

The light sources 124 suitable for use can include various types of light sources including lasers and lamps. For example, in certain non-exclusive embodiments, the light source 124 can be an infrared laser that emits light energy in the form of pulses of infrared light. Alternatively, as noted above, the light sources 124, as referred to herein, can include any suitable type of energy source.

Suitable lasers can include short pulse lasers on the sub-millisecond timescale. In some embodiments, the light source 124 can include lasers on the nanosecond (ns) timescale. The lasers can also include short pulse lasers on the picosecond (ps), femtosecond (fs), and microsecond (us) timescales. It is appreciated that there are many combinations of laser wavelengths, pulse widths, and energy levels that can be employed to achieve plasma in the balloon fluid 132 of the catheter 102. In various non-exclusive alternative embodiments, the pulse widths can include those falling within a range including from at least ten ns to 3000 ns, at least 20 ns to 100 ns, or at least one ns to 500 ns. Alternatively, any other suitable pulse width range can be used.

Exemplary nanosecond lasers can include those within the UV to IR spectrum, spanning wavelengths of about ten nanometers (nm) to one millimeter (mm). In some embodiments, the light sources 124 suitable for use in the catheter system 100 can include those capable of producing light at wavelengths of from at least 750 nm to 2000 nm. In other embodiments, the light sources 124 can include those capable of producing light at wavelengths of from at least 700 nm to 3000 nm. In still other embodiments, the light sources 124 can include those capable of producing light at wavelengths of from at least 100 nm to ten micrometers (µm). Nanosecond lasers can include those having repetition rates of up to 200 kHz. In some embodiments, the laser can include a Q-switched thulium:yttrium-aluminum-garnet (Tm:YAG) laser. In other embodiments, the laser can include a neodymium:yttrium-aluminum-garnet (Nd:YAG) laser, holmium:yttrium-aluminum-garnet (Ho:YAG) laser, erbium:yttrium-aluminum-garnet (Er:YAG) laser, excimer laser, helium-neon laser, carbon dioxide laser, as well as doped, pulsed, fiber lasers.

The catheter system 100 can generate pressure waves having maximum pressures in the range of at least one megapascal (MPa) to 100 MPa. The maximum pressure generated by a particular catheter system 100 will depend on the light source 124, the absorbing material, the bubble expansion, the propagation medium, the balloon material, and other factors. In various non-exclusive alternative embodiments, the catheter system 100 can generate pressure waves having maximum pressures in the range of at least approximately two MPa to 50 MPa, at least approximately two MPa to 30 MPa, or at least approximately 15 MPa to 25 MPa.

The pressure waves can be imparted upon the treatment site 106 from a distance within a range from at least approximately 0.1 millimeters (mm) to greater than approximately 25 mm extending radially from the light guides 122A when the catheter 102 is placed at the treatment site 106. In various non-exclusive alternative embodiments, the pressure waves can be imparted upon the treatment site 106 from a distance within a range from at least approximately ten mm to 20 mm, at least approximately one mm to ten mm, at least approximately 1.5 mm to four mm, or at least approximately 0.1 mm to ten mm extending radially from the light guides 122A when the catheter 102 is placed at the treatment site 106. In other embodiments, the pressure waves can be imparted upon the treatment site 106 from another suitable distance that is different than the foregoing ranges. In some embodiments, the pressure waves can be imparted upon the treatment site 106 within a range of at least approximately two MPa to 30 MPa at a distance from at least approximately 0.1 mm to ten mm. In some embodiments, the pressure waves can be imparted upon the treatment site 106 from a range of at least approximately two MPa to 25 MPa at a distance from at least approximately 0.1 mm to ten mm. Still alternatively, other suitable pressure ranges and distances can be used.

The power source 125 is electrically coupled to and is configured to provide the necessary power to each of the light source 124, the system controller 126, the GUI 127, and the handle assembly 128. The power source 125 can have any suitable design for such purposes.

The system controller 126 is electrically coupled to and receives power from the power source 125. Additionally, the system controller 126 is coupled to and is configured to control the operation of each of the light source 124, and the GUI 127. The system controller 126 can include one or more processors or circuits for purposes of controlling the operation of at least the light source 124, and the GUI 127. For example, the system controller 126 can control the light source 124 for generating pulses of light energy as desired and/or at any desired firing rate.

The system controller 126 can further be configured to control the operation of other components of the catheter system 100 such as the positioning of the catheter 102 adjacent to the treatment site 106, the inflation of the balloon 104 with the balloon fluid 132, etc. Further, or in the alternative, the catheter system 100 can include one or more additional controllers that can be positioned in any suitable manner for purposes of controlling the various operations of the catheter system 100. For example, in certain embodiments, an additional controller and/or a portion of the system controller 126 can be positioned and/or incorporated within the handle assembly 128.

The GUI 127 is accessible by the user or operator of the catheter system 100. Additionally, the GUI 127 is electrically connected to the system controller 126. With such design, the GUI 127 can be used by the user or operator to ensure that the catheter system 100 is effectively utilized to impart pressure onto and induce fractures at the treatment site(s) 106. The GUI 127 can provide the user or operator with information that can be used before, during, and after use of the catheter system 100. In one embodiment, the GUI 127 can provide static visual data and/or information to the user or operator. In addition, or in the alternative, the GUI 127 can provide dynamic visual data and/or information to the user or operator, such as video data or any other data that changes over time during the use of the catheter system 100. In various embodiments, the GUI 127 can include one or more colors, different sizes, varying brightness, etc., that may act as alerts to the user or operator. Additionally, or in the alternative, the GUI 127 can provide audio data or information to the user or operator. The specifics of the GUI 127 can vary depending upon the design requirements of the catheter system 100, or the specific needs, specifications, and/or desires of the user or operator.

As shown in Figure 1, the handle assembly 128 can be positioned at or near the proximal portion 114 of the catheter system 100, and/or near the source manifold 136. In this embodiment, the handle assembly 128 is coupled to the balloon 104 and is positioned spaced apart from the balloon 104. Alternatively, the handle assembly 128 can be positioned at another suitable location.

The handle assembly 128 is handled and used by the user or operator to operate, position, and control the catheter 102. The design and specific features of the handle assembly 128 can vary to suit the design requirements of the catheter system 100. In the embodiment illustrated in Figure 1, the handle assembly 128 is separate from, but in electrical and/or fluid communication with one or more of the system controller 126, the light source 124, the fluid pump 138, and the GUI 127. In some embodiments, the handle assembly 128 can integrate and/or include at least a portion of the system controller 126 within an interior of the handle assembly 128. For example, as shown, in certain such embodiments, the handle assembly 128 can include circuitry 156 that can form at least a portion of the system controller 126.

In one embodiment, the circuitry 156 can include a printed circuit board having one or more integrated circuits, or any other suitable circuitry. In an alternative embodiment, the circuitry 156 can be omitted or can be included within the system controller 126, which in various embodiments can be positioned outside of the handle assembly 128, e.g., within the multiplexer 123. It is understood that the handle assembly 128 can include fewer or additional components than those specifically illustrated and described herein.

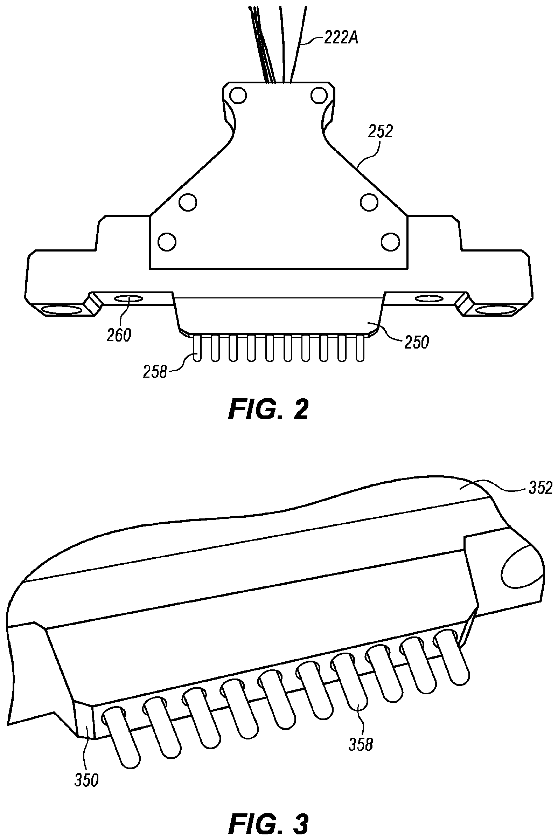

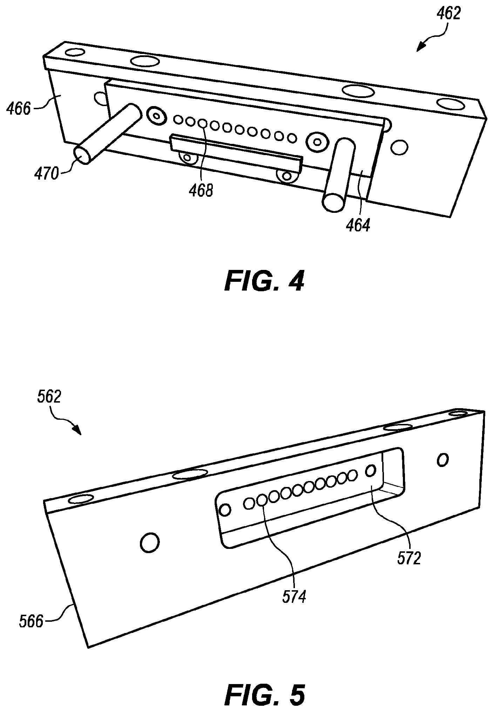

Figure 2 is a top view of a portion of an embodiment of the catheter system 100 (illustrated in Figure 1) including an embodiment of a guide bundle 252. As illustrated in the embodiment in Figure 2, the guide coupling housing 250 houses one or more ferrule assemblies 258 and one or more alignment guide receivers 260. The ferrule assemblies 258 can each include a ferrule 778 (illustrated in Figure 7, for example), a portion of the individual light guide 222A, including the guide proximal end 122P (illustrated in Figure 1, for example), and a spring 776 (illustrated in Figure 7, for example). The ferrule assembly 258 can retain the ferrule 778. In certain embodiments, the light guide 222A can be engaged by a ferrule assembly 258. In particular, the guide proximal end 122P can be terminated into individual ferrules 778 of the ferrule assembly 258. The ferrule assembly 258 can be configured to provide increased localization and improved alignment guides that enable each ferrule 778 to be inserted into receptacle holes 468 (illustrated in Figure 4, for example) within the receptacle assembly 462 (illustrated in Figure 4, for example) without damaging the ferrules 778.

The ferrule assembly 258 can vary depending on the design requirements of the catheter system 100, the light guide 222A, the guide proximal end 122P, and/or guide bundle 252. It is understood that the ferrule assembly 258 can include additional systems, subsystems, components, and elements than those specifically shown and/or described herein. Additionally, or alternatively, the ferrule assembly 258 can omit one or more of the systems, subsystems, and elements that are specifically shown and/or described herein. The ferrule assembly 258 can be positioned in any suitable location, including those shown in Figure 2.

The ferrule assembly 258 can vary in shape. In some embodiments, the ferrule assembly 258 can be substantially plug-shaped, as shown in the embodiment illustrated in Figure 2. In other embodiments, the ferrule assembly 258 can be substantially cylindrical-shaped, prism-shaped, cube-shaped, cuboid-shaped, and/or tube-shaped.

The ferrule assembly 258 can be formed with any suitable material. In certain embodiments, the ferrule assembly 258 can be at least partially formed from a metal, a plastic, a polymer, a ceramic, a composite, and/or an organic material. In other embodiments, the ferrule assembly 258 can be formed using mold-injected plastics.

The alignment guide receiver 260 receives an alignment guide 470 (illustrated in Figure 4, for example). The alignment guide receiver 260 can be configured to improve the mating of the ferrule 778 with the receptacle hole 468 within the receptacle assembly 462.

The alignment guide receiver 260 can vary depending on the design requirements of the catheter system 100, the guide bundle 252, and/or the alignment guide 470. It is understood that the alignment guide receiver 260 can include additional systems, subsystems, components, and elements than those specifically shown and/or described herein. Additionally, or alternatively, the alignment guide receiver 260 can omit one or more of the systems, subsystems, and elements that are specifically shown and/or described herein. The alignment guide receiver 260 can be positioned in any suitable location, including those shown in Figure 2.

In the embodiment shown in Figure 2, the alignment guide receiver 260 is a pin receiver. Non-limiting, non-exclusive examples of suitable alignment guide receivers include (i) a guide groove of a tongue and groove system, (ii) a guide slot of a tab and slot system, and (iii) a guide channel of a rail and channel system.

Figure 3 is a perspective front view of a portion of an embodiment of the catheter system 100 (illustrated in Figure 1) including an embodiment of a guide bundle 352. In particular, greater detail of the shape and design of the embodiments of the guide coupling housing 350, the guide bundle 352, and the ferrule assembly 358 are illustrated in Figure 3. For example, as shown in Figure 3, the guide coupling housing 350 can have a substantially trapezoidal side-profile and a substantially trapezoidal-prism shape. The guide coupling housing 350 can extend outwardly from the guide bundle 352. The guide coupling housing 350 can include one or more apertures, each of the ferrule assemblies 358 can extend through corresponding apertures. As illustrated in the embodiment in Figure 3, the ferrule assemblies 358 can be substantially cylindrical with a partially spherical end portion. The ferrule assemblies 358 can include an opening or orifice in order to receive the guide beams 122B (illustrated in Figure 1) from the multiplexer 128 (Illustrated in Figure 1).

Figure 4 is a perspective front view of a portion of an embodiment of the catheter system 100 (illustrated in Figure 1) including an embodiment of a receptacle assembly 462. As illustrated in the embodiment in Figure 4, the receptacle assembly 462 can include one or more of a receptacle block 464, a receptacle housing 466, a receptacle hole 468, and an alignment guide 470. The receptacle assembly 462 can be configured to mate with the guide bundle 252 (illustrated in Figure 2, for example) so that the guide beams 122B are aligned with each of the light guides 222A (illustrated in Figure 2, for example) within the guide bundle 252.

The receptacle assembly 462 can vary depending on the design requirements of the catheter system 100, the light guides 222A, the guide bundle 252 and/or the ferrule assembly 358 (illustrated in Figure 3, for example). It is understood that the receptacle assembly 462 can include additional systems, subsystems, components, and elements than those specifically shown and/or described herein. Additionally, or alternatively, the receptacle assembly 462 can omit one or more of the systems, subsystems, and elements that are specifically shown and/or described herein. The receptacle assembly 462 can be positioned in any suitable location, including those shown in Figure 4.

The receptacle assembly 462 can be formed from any suitable material. In some embodiments, the components of the receptacle assembly 462 can be formed at least partially from a metal, a plastic, a ceramic, a polymer, a composite, and/or an organic material. In certain embodiments, the components of the receptacle assembly 462 can be formed from a hardened stainless steel configured to have increased resilience and resistance to wear and tear.

The receptacle block 464 can be configured to receive the guide bundle 252. The receptacle block 464 can vary depending on the design requirements of the catheter system 100, the light guides 222A, the guide bundle 252, and/or the receptacle assembly 462. It is understood that the receptacle block 464 can include additional systems, subsystems, components, and elements than those specifically shown and/or described herein. Additionally, or alternatively, the receptacle block 464 can omit one or more of the systems, subsystems, and elements that are specifically shown and/or described herein. The receptacle block 464 can be positioned in any suitable location, including those shown in Figure 4.

The receptacle housing 466 can house the receptacle block 464. In some embodiments, the receptacle housing 466 can be coupled to the receptacle block 464. The receptacle housing 466 can vary depending on the design requirements of the catheter system 100, the light guides 222A, the guide bundle 252, the receptacle assembly 462, and/or the receptacle block 464. It is understood that the receptacle housing 466 can include additional systems, subsystems, components, and elements than those specifically shown and/or described herein. Additionally, or alternatively, the receptacle housing 466 can omit one or more of the systems, subsystems, and elements that are specifically shown and/or described herein. The receptacle housing 466 can be positioned in any suitable location, including those shown in Figure 4.

The receptacle hole 468 can be precision-bored into the receptacle block 464. Alternatively, the receptacle hole 468 can be formed in the receptacle block 464 by any suitable method known in the art. As shown in the embodiment in Figure 4, one or more receptacle holes 468 can be aligned in a linear array in the receptacle block 464. The receptacle hole 468 can vary depending on the design requirements of the catheter system 100, the light guides 222A, the guide bundle 252, the receptacle assembly 462, and/or the receptacle block 464. It is understood that the receptacle hole 468 can include additional systems, subsystems, components, and elements than those specifically shown and/or described herein. Additionally, or alternatively, the receptacle hole 468 can omit one or more of the systems, subsystems, and elements that are specifically shown and/or described herein.

The receptacle hole 468 can be positioned in any suitable location, including those shown in Figure 4. The receptacle assembly 462 can include any suitable number of receptacle holes 468 such as 1, 2, 3, 4, 5, 7, 8, 9, or 10 receptacle holes 468. In other embodiments, the receptacle assembly 462 can include greater than 10 receptacle holes 468.

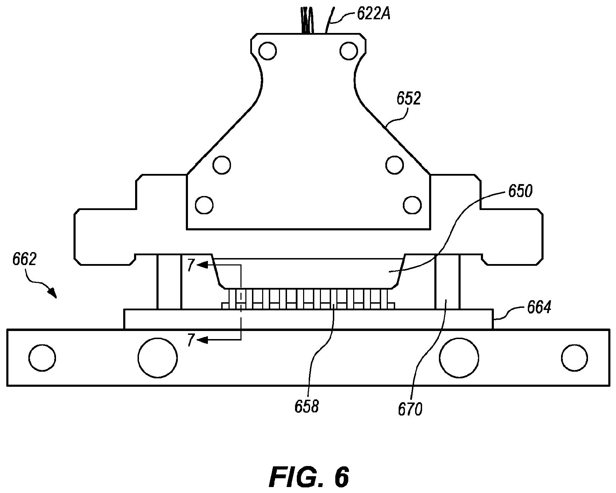

The alignment guide 470 can be coupled to the receptacle block 464. The alignment guide 470 can mate with the alignment guide receiver 260 (illustrated in Figure 2) so that the guide bundle 252 and the receptacle block 464 are at least partially aligned and/or coupled (for example, as illustrated in Figure 6). The alignment guide 470 can vary depending on the design requirements of the catheter system 100, the light guides 222A, the guide bundle 252, the alignment guide receiver 260, the receptacle assembly 462, and/or the receptacle block 464. It is understood that the alignment guide 470 can include additional systems, subsystems, components, and elements than those specifically shown and/or described herein. Additionally, or alternatively, the alignment guide 470 can omit one or more of the systems, subsystems, and elements that are specifically shown and/or described herein.

The alignment guide 470 can be positioned in any suitable location, including those shown in Figure 4. The alignment guide 470 can be substantially dowel-shaped. The receptacle assembly 462 can include any suitable number of alignment guides 470 such as 1, 2, 3, 4, 5, 7, 8, 9, or 10 alignment guides 470. In other embodiments, the receptacle assembly 462 can include greater than 10 alignment guides 470. The alignment guide 470 can be any suitable alignment guide 470 known within the art. In the embodiment shown in Figure 4, the alignment guide 470 is a guide pin. Non-limiting, non-exclusive examples of suitable alignment guides include (i) a guide tongue of a tongue and groove system, (ii) a guide tab of a tab and slot system, and (iii) a guide rail of a rail and channel system.

Figure 5 is a perspective rear view of a portion of an embodiment of the catheter system 100 (illustrated in Figure 1) including an embodiment of a receptacle assembly 562. As illustrated in the embodiment displayed in Figure 5, the receptacle assembly 562 can include a receptacle housing 566 and a backing plate 572 including one or more alignment holes 574.

The backing plate 572 can be configured to receive a proximal end face of the ferrules 778. The backing plate 572 can provide a precision reference plane for aligning the guide beams 122B from the multiplexer 128 to each of the corresponding light guides 122A (illustrated in Figure 1). The alignment holes 574 can be precision-bored into the backing plate 572. The alignment holes 574 can be formed into the backing plate 572 using any suitable method known in the art. The alignment holes 574 can be configured to couple the focused guide beams 122B with each of the corresponding light guides 122A.

The backing plate 572 and alignment holes 574 can vary depending on the design requirements of the catheter system 100, the light guides 222A, the guide bundle 252, the alignment guide receiver 260, the receptacle assembly 462, and/or the receptacle block 464. It is understood that the backing plate 572 can include additional systems, subsystems, components, and elements than those specifically shown and/or described herein. Additionally, or alternatively, the backing plate 572 can omit one or more of the systems, subsystems, and elements that are specifically shown and/or described herein. The backing plate 572 and alignment holes 574 can be positioned in any suitable location, including those shown in Figure 5. In certain embodiments, the alignment holes 574 can have a smaller diameter than the diameter of the ferrule 778. The alignment holes 574 can have a sufficiently-sized diameter in order to accommodate the entire guide beam 128B without clipping.

Figure 6 is a top view of a portion of an embodiment of the catheter system 100 (illustrated in Figure 1) including an embodiment of a guide bundle 652 including light guides 622A and a receptacle assembly 662, the guide bundle 652 being shown in a partially connected state with respect to the receptacle assembly 662. In particular, in the embodiment illustrated in Figure 6, the guide coupling housing 650 is advanced so that the ferrule assemblies 658 and the alignment guides 670 are partially seated within the receptacle block 664.

Figure 7 is a simplified cross-sectional view of a portion of an embodiment of the catheter system 100 (illustrated in Figure 1) taken on line 7-7 in Figure 6, the catheter system 100 including an embodiment of a guide coupling housing 750 including light guides 722A and a receptacle assembly 762, the guide coupling housing 750 being shown in a partially connected state with respect to the receptacle assembly 762. In particular, in the embodiment illustrated in Figure 7, the guide coupling housing 750 is advanced so that the ferrule assemblies 758 and the alignment guides 770 are partially seated within the receptacle block 764. In Figure 7, the ferrule assembly 758 is (i) partially extended through the receptacle hole 768, and (ii) partially engaged with the backing plate 772 of the receptacle assembly 762 so that the alignment hole 774 is aligned with the ferrule assembly 758. As illustrated in the embodiment displayed in Figure 7, the ferrule assembly 758 can further include a spring 776, a ferrule 778, and a ferrule collar 780.

The spring 776 can engage the ferrule 778 and/or the ferrule collar 780 within the ferrule assembly 758. The spring 776 can be configured to provide an insertion force when the ferrule assembly 758 is inserted into the receptacle hole 768. The spring 776 can vary depending on the design requirements of the catheter system 100, the light guides 222A, the guide coupling housing 750, the receptacle assembly 762, the receptacle hole 768, the ferrule 778, and/or the ferrule collar 780. It is understood that the spring 776 can include additional systems, subsystems, components, and elements than those specifically shown and/or described herein. Additionally, or alternatively, the spring 776 can omit one or more of the systems, subsystems, and elements that are specifically shown and/or described herein. The spring 776 can be positioned in any suitable location, including those shown in Figure 7. The spring 776 can be a spiral spring.

The ferrule 778 can engage the guide proximal end 122P (illustrated in Figure 1) of the light guide 722A within the ferrule assembly 758. The ferrule 778 can fasten, reinforce, and/or seal the guide proximal end 122P of the light guide 722A. The ferrule 778 can vary depending on the design requirements of the catheter system 100, the light guides 722A, the guide coupling housing 750, the receptacle assembly 762, the receptacle hole 768, the spring 776, and/or the ferrule collar 780. It is understood that the ferrule 778 can include additional systems, subsystems, components, and elements than those specifically shown and/or described herein. Additionally, or alternatively, the ferrule 778 can omit one or more of the systems, subsystems, and elements that are specifically shown and/or described herein. The ferrule 778 can be positioned in any suitable location, including those shown in Figure 7.

The ferrule 778 can be formed from any suitable material. In certain embodiments, the ferrule 778 can be partially formed from a metal, a plastic, a ceramic, a polymer, a composite, and/or an organic material. The ferrule 778 can have a proximal end face 778P. In some embodiments, the proximal end face 778P can engage the backing plate 772 when the ferrule 778 is at least partially inserted in the receptacle hole 768. As shown in Figure 7, the alignment hole 774 can have a smaller diameter than the ferrule 778 so that the proximal end face 778P of the ferrule 778 is seated on the alignment hole 774.

The ferrule collar 780 can back the ferrule 778. The ferrule collar 780 can be seated within the spring 776. The ferrule 778 can vary depending on the design requirements of the catheter system 100, the light guides 222A, the guide coupling housing 750, the receptacle assembly 762, the receptacle hole 768, the spring 776, and/or the ferrule collar 780. It is understood that the ferrule 778 can include additional systems, subsystems, components, and elements than those specifically shown and/or described herein. Additionally, or alternatively, the ferrule 778 can omit one or more of the systems, subsystems, and elements that are specifically shown and/or described herein. The ferrule 778 can be positioned in any suitable location, including those shown in Figure 7.

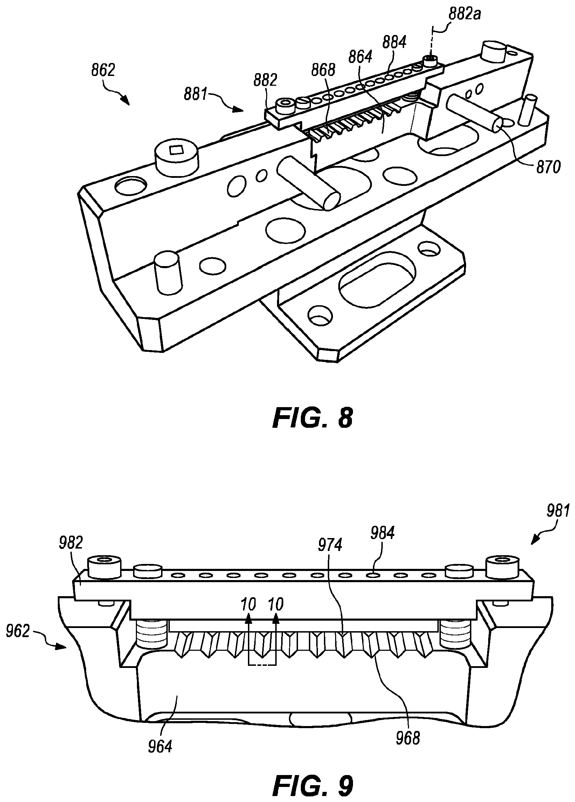

Figure 8 is a perspective front view of a portion of an embodiment of the catheter system 100 (illustrated in Figure 1) including an embodiment of a receptacle assembly 862. Figure 8 illustrates one embodiment of the receptacle assembly 862 that is slightly different than the other embodiments described herein. In the embodiment illustrated in Figure 8, the receptacle block 864 includes one or more receptacle holes 868 that are formed in a linear array as v-grooves, rather than singular holes.

In some embodiments, the v-groove receptacle holes 868 shown in Figure 8 can be formed by cutting into a hardened block of stainless steel. The v-grooves can be electrical discharge machine cut into the receptacle holes 868. The v-grooves provide at least two contact lines while the ferrule 778 is retained within the receptacle hole 868. In the embodiment illustrated in Figure 8, the receptacle assembly 862 can include a retainer assembly 881 including a clamping bar 882 having a clamping bar axis 882a, and a plunger 884. The retaining assembly 881 retains the ferrule 778 within the receptacle hole 868. The retaining assembly 881 can be configured to reduce and/or inhibit the insertion forces required to retain the ferrule 778 within the receptacle hole 868.

The retaining assembly 881 can vary depending on the design requirements of the catheter system 100, the receptacle assembly 862, and/or the ferrule 778. It is understood that the retaining assembly 881 can include additional systems, subsystems, components, and elements than those specifically shown and/or described herein. Additionally, or alternatively, the retaining assembly 881 can omit one or more of the systems, subsystems, and elements that are specifically shown and/or described herein. The retaining assembly 881 can be positioned in any suitable location, including those shown in Figure 8.

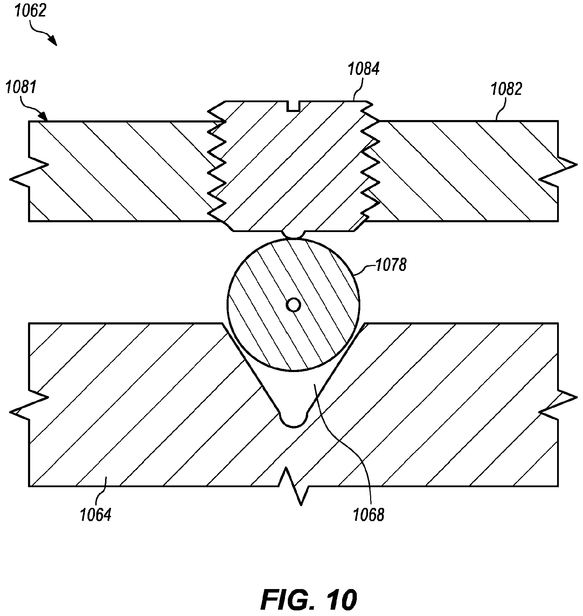

The clamping bar 882 clamps the ferrule 778 into the receptacle hole 868. The clamping bar 882 can be moved about the clamping bar axis 882a to apply and/or remove a retaining force onto one or more ferrule 778 (for example, see Figure 10). In certain embodiments, the clamping bar 882 can be raised and lowered thereby allowing (i) movement of the ferrule 778 upon insertion, and (ii) retention of the ferrule 778 once the proximal end face 778P is seated against the backing plate 772 (illustrated in Figure 7) and the clamping bar 882 is lowered to retain the ferrule 778.

The clamping bar 882 can vary depending on the design requirements of the catheter system 100, the receptacle assembly 862, the ferrule 778, the retaining assembly 881, and/or the plunger 884. It is understood that the clamping bar 882 can include additional systems, subsystems, components, and elements than those specifically shown and/or described herein. Additionally, or alternatively, the clamping bar 882 can omit one or more of the systems, subsystems, and elements that are specifically shown and/or described herein. The clamping bar 882 can be positioned in any suitable location, including those shown in Figure 8.

The clamping bar 882 can include a compliant material and/or wave springs that are configured to spread a retaining force across the ferules 778 so that each of the ferrules 778 is retained within a corresponding receptacle hole 868. The clamping bar 882 can retain the ferrule 778 so that the ferrule 778 is not free-floating within the receptacle hole 868. The clamping bar 882 can also be configured to provide a uniform or substantially uniform retaining force to the ferrules 778, as known in the art.

The plunger 884 engages (e.g., plunges) the ferrule 778 into the receptacle hole 868 so that the ferrule 778 is retained in place. In some embodiments, the plunger 884 engages the upper edges of the ferrule 778, seating it into the v-groove of the receptacle hole 868. The plunger 884 can vary depending on the design requirements of the catheter system 100, the receptacle assembly 862, the ferrule 778, the retaining assembly 881, and/or the clamping bar 882. It is understood that the plunger 884 can include additional systems, subsystems, components, and elements than those specifically shown and/or described herein. Additionally, or alternatively, the plunger 884 can omit one or more of the systems, subsystems, and elements that are specifically shown and/or described herein.

The plunger 884 can be positioned in any suitable location, including those shown in Figure 8. The plunger 884 can be substantially similar in form and/or function as a ballspring plunger. The plunger 884 can be integrated into the clamping bar 882 (for example, as illustrated in Figure 8).

Figure 9 is a front elevation view of a portion of an embodiment of the catheter system 100 (illustrated in Figure 1) including an embodiment of a portion of a receptacle assembly 962. Figure 9 illustrates one embodiment of the receptacle assembly 962 that is slightly different than the other embodiments described herein. In the embodiment illustrated in Figure 9, greater detail is shown of the interior of the receptacle block 964 including a receptacle hole 968. In Figure 9, the alignment hole 974 and the retaining assembly 981 including the clamping bar 982 and the plunger 984 are shown.

Figure 10 is a simplified cross-sectional view of a portion of an embodiment of the catheter system 100 (illustrated in Figure 1) taken on line 10-10 in Figure 9, the catheter system 100 including an embodiment of a receptacle assembly 1062. Figure 10 illustrates one embodiment of the receptacle assembly 1062 that is slightly different than the other embodiments described herein. In the embodiment illustrated in Figure 10, the receptacle block 1064 includes one or more receptacle holes 1068 that are formed in a linear array as v-grooves, rather than singular holes. In the embodiment illustrated in Figure 10, the receptacle assembly 1062 can include a retainer assembly 1081 including a clamping bar 1082, and a plunger 1084. The retaining assembly 1081 retains the ferrule 1078 within the receptacle hole 1068. The retaining assembly 1081 can be configured to reduce and/or inhibit the insertion forces required to retain the ferrule 1078 within the receptacle hole 1068. The clamping bar 1082 clamps the ferrule 1078 into the receptacle hole 1068.

This technology provides a connector solution for multiple optical channels that improves optical coupling to an individual light guide organized in a multi-channel array. The technology can utilize individual, low-cost ferrules to terminate the light guides. These ferrules are carried in ferrule assembly included within the guide bundle. In some embodiments, the guide bundle aligns the ferrules into a linear array. The ferrules can float within the ferrule assembly with low location tolerances. The connectorized ferrule assembly can mate with a high-precision receptacle assembly including a receptacle plate and/or receptacle block. Mechanical features in the receptacle assembly capture and align the individual ferrules. These features can align the floating ferrules in an improved precision array with tightly controlled tolerances. This technology allows a single, stable energy source to be channeled sequentially through a plurality of channels with a variable number.

Specific advantages this technology provides include: 1) enabling the use of low cost, high-precision, singular ferrules on a single-use device for an increased-reliability connection thereby reducing production costs, 2) reducing the system performance dependence on the assembly of light guides into a ferrule block and the associated mechanical tolerances related to their location in a multi-channel array, 3) reducing the performance dependence on accuracy of connecting and aligning the multi-channel array to the multiplexer, 4) reducing the need for a high-cost, high-precision monolithic ferrule block.

In some embodiments the light guides are optical fibers, the energy source is a pulsed laser and the emitters are plasma generators. In its simplest form, the multiplexer is a precision linear mechanism that translates coupling optics along a linear path. This approach requires a single degree of freedom. A connector block organizes the individual optical fibers into a liner pattern with precise interval spacing.

In certain embodiments, the linear translation mechanism is electronically controlled by the system to line the beam path up sequentially with each individual fiber organized in the ferrule. The translating mechanism carries necessary beam directing optics and focusing optics to focus the laser energy onto each fiber for optimal coupling. That way, the low divergence of the laser beam over the short distance of motion of the translated coupling mechanism has a minimum impact on coupling efficiency to the fiber. The system drives the mechanism to align the beam path with a selected fiber optic channel and then fires the laser in pulsed or semi-continuous wave mode.

This system and method can be implemented for any multiplexer configuration either linear, circular, patterned, or scanned. In some embodiments, the system can include a probe and a plurality of primary laser beams that can be combined and spot traced by beam paths that are correlated to the parametric motion of the multiplexer mechanism.

It is appreciated that the systems and methods of optical alignment provided herein address multiple potential issues with the performance, reliability, and proper usage of an IVL catheter, in particular one that utilizes an energy source to create a localized plasma which in turn produces a high energy bubble inside a balloon. Specific problems solved by the systems and methods disclosed herein include:
1) The technology disclosed herein reduces the optical coupling dependence on the precision and mechanical tolerance stack-ups of assemblies and true alignment for light guides (such as optical fibers), ferrules, connectors, and receptacles, enabling the use of low-cost, low-precision components on the single-use device and improve the cost of goods sold.
2) The technology disclosed herein reduces the multiplexer performance dependence on the accuracy of positioning mechanism in the multiplexer and associated quality and precision of its optical and mechanical components thereby improving speed and performance of the multiplexer and multi-channel ferrule system.

It should be noted that, as used in this specification and the appended claims, the singular forms "a," "an," and "the" include plural referents unless the content and/or context clearly dictates otherwise. It should also be noted that the term "or" is generally employed in its sense including "and/or" unless the content or context clearly dictates otherwise.

It should also be noted that, as used in this specification and the appended claims, the phrase "configured" describes a system, apparatus, or other structure that is constructed or configured to perform a particular task or adopt a particular configuration. The phrase "configured" can be used interchangeably with other similar phrases such as arranged and configured, constructed and arranged, constructed, manufactured and arranged, and the like.

The headings used herein are provided for consistency with suggestions under 37 CFR 1.77 or otherwise to provide organizational cues. These headings shall not be viewed to limit or characterize the invention(s) set out in any claims that may issue from this disclosure. As an example, a description of a technology in the "Background" is not an admission that technology is prior art to any invention(s) in this disclosure. Neither is the "Summary" or "Abstract" to be considered as a characterization of the invention(s) set forth in issued claims.

The embodiments described herein are not intended to be exhaustive or to limit the invention to the precise forms disclosed in the detailed description provided herein. Rather, the embodiments are chosen and described so that others skilled in the art can appreciate and understand the principles and practices. As such, aspects have been described with reference to various specific and preferred embodiments and techniques.

It is understood that although a number of different embodiments of the catheter systems have been illustrated and described herein, one or more features of any one embodiment can be combined with one or more features of one or more of the other embodiments, provided that such combination satisfies the intent of the present invention.

While a number of exemplary aspects and embodiments of the catheter systems have been discussed above, those of skill in the art will recognize certain modifications, permutations, additions, and sub-combinations thereof. It is therefore intended that the following appended claims and claims hereafter introduced are interpreted to include all such modifications, permutations, additions, and sub-combinations as are within their scope, and no limitations are intended to the details of construction or design herein shown.

## Claims

1. A catheter system (100) for treating a treatment site (106) within or adjacent to a vessel wall (108A) or a heart valve, the catheter system (100) comprising:
a light source (124) that generates light energy;
a first light guide (122A, 222A, 622A, 722A) that receives the light energy from the light source (124), the first light guide (122A, 222A, 622A, 722A) having a guide proximal end (122P);
a second light guide that receives the light energy from the light source (124), the second light guide having a guide proximal end (122P);
a guide bundle (122, 152, 252, 352, 652) that is in optical communication with the light source (124), the guide bundle (122, 152, 252, 352, 652) bundling the first light guide (122A, 222A, 622A, 722A) and the second light guide, the guide bundle (122, 152, 252, 352, 652) including a first ferrule (778, 1078) that engages the guide proximal end (122P) of the first light guide (122A, 222A, 622A, 722A) and a second ferrule that engages the guide proximal end (122P) of the second light guide; and
a receptacle assembly (462, 562, 662, 762, 862, 962, 1062) including (i) a first receptacle hole (468, 768, 868, 968, 1068) that is configured to receive the first ferrule (778, 1078), (ii) a second receptacle hole that is configured to receive the second ferrule, (iii) a receptacle block (464, 664, 764, 864, 964, 1064), and (iv) a backing plate (572, 772) that is coupled to the receptacle block (464, 664, 764, 864, 964, 1064), the backing plate (572, 772) being configured to engage the first ferrule (778, 1078) and the second ferrule
wherein the first receptacle hole (468, 768, 868, 968, 1068) and the second receptacle hole are each formed in the receptacle block (464, 664, 764, 864, 964, 1064) as v-grooves.

2. The catheter system (100) of claim 1 wherein the guide bundle (122, 152, 252, 352, 652) further includes (i) a first ferrule assembly (258, 358, 658, 758) including the first ferrule (778, 1078) and a first spring (776) that engages the first ferrule (778, 1078), the first spring (776) being configured to provide an insertion force when the first ferrule (778, 1078) is inserted into the first receptacle hole (468, 768, 868, 968, 1068), and (ii) a second ferrule assembly including the second ferrule and a second spring that engages the second ferrule, the second spring being configured to provide an insertion force when the second ferrule is inserted into the second receptacle hole.

3. The catheter system (100) of any one of claims 1-2 wherein at least one of the first ferrule (778, 1078) and the second ferrule are formed at least partially from a ceramic material.

4. The catheter system (100) of any one of claims 1-3 wherein at least one of the first ferrule (778, 1078) and the second ferrule are formed at least partially from a metallic material.

5. The catheter system (100) of any one of claims 1-4 wherein the receptacle assembly (462, 562, 662, 762, 862, 962, 1062) is formed at least partially from at least one of a ceramic material and a metallic material.

6. The catheter system (100) of any one of claims 1-5 further comprising an alignment guide (470, 670, 770) and an alignment guide receiver (260), the alignment guide receiver (260) being configured to receive the alignment guide (470, 670, 770) and to improve mating of the first ferrule (778, 1078) with the first receptacle hole (468, 768, 868, 968, 1068) and the second ferrule with the second receptacle hole.

7. The catheter system (100) of any one of claims 1-6 wherein each of the ferrules (766, 778, 1078) includes a proximal end face (778P), and wherein the backing plate (572, 772) is configured to engage each of the proximal end faces (778P).

8. The catheter system (100) of any one of claims 1-7 wherein the backing plate (572, 772) includes (i) a first alignment hole that is configured to align a first guide beam with the first light guide (122A, 222A, 622A, 722A), and (ii) a second alignment hole that is configured to align a second guide beam with the second light guide.

9. The catheter system (100) of any one of claims 1-8 wherein the first receptacle hole (468, 768, 868, 968, 1068) and the second receptacle hole are each formed in the receptacle block (464, 664, 764, 864, 964, 1064), the first receptacle hole (468, 768, 868, 968, 1068) and the second receptacle hole being aligned on a same linear axis as one another.

10. The catheter system (100) of any one of claims 1-9 wherein the receptacle assembly (462, 562, 662, 762, 862, 962, 1062) further includes a retainer assembly (881, 1081) that is configured to retain (i) the first ferrule (778, 1078) in the first receptacle hole (468, 768, 868, 968, 1068), and (ii) the second ferrule in the second receptacle hole.

11. The catheter system (100) of claim 10 wherein the retainer assembly (881, 1081) includes a clamping bar (882, 982, 1082) having a ball spring plunger, the ball spring plunger being configured to contact (i) at least a portion (114, 116) of the first ferrule (778, 1078) so that the first ferrule (778, 1078) is retained in the first receptacle hole (468, 768, 868, 968, 1068), and (ii) at least a portion (114, 116) of the second ferrule so that the second ferrule is retained in the second receptacle hole.

12. The catheter system (100) of claim 11 wherein the plunger (884, 984, 1084) is integrated into the clamping bar (882, 982, 1082).

13. The catheter system (100) of any one of claims 11-12 wherein the clamping bar (882, 982, 1082) is configured to be rotatable about a clamping bar axis (882a), the clamping bar (882, 982, 1082) including a compliant material that is configured to spread a retaining force across the ferrules (766, 778, 1078) so that each ferrule (766, 778, 1078) is retained within a corresponding receptacle hole.

14. The catheter system (100) of any one of claims 1-13 wherein the light source (124) includes a laser, and wherein each of the first light guide (122A, 222A, 622A, 722A) and the second light guide includes an optical fiber.

## Patentansprüche

1. Kathetersystem (100) zum Behandeln einer Behandlungsstelle (106) innerhalb oder benachbart zu einer Gefäßwand (108A) oder einer Herzklappe, wobei das Kathetersystem (100) aufweist:
eine Lichtquelle (124), die Lichtenergie erzeugt;
einen ersten Lichtleiter (122A, 222A, 622A, 722A), der die Lichtenergie von der Lichtquelle (124) empfängt, wobei der erste Lichtleiter (122A, 222A, 622A, 722A) ein proximales Leiterende (122P) aufweist;
einen zweiten Lichtleiter, der die Lichtenergie von der Lichtquelle (124) empfängt, wobei der zweite Lichtleiter ein proximales Leiterende (122P) aufweist;
ein Lichtleiterbündel (122, 152, 252, 352, 652), das mit der Lichtquelle (124) in optischer Kommunikation steht, wobei das Lichtleiterbündel (122, 152, 252, 352, 652) den ersten Lichtleiter (122A, 222A, 622A, 722A) und den zweiten Lichtleiter bündelt, wobei das Lichtleiterbündel (122, 152, 252, 352, 652) eine erste Ferrule (778, 1078) aufweist, die mit dem proximalen Leiterende (122P) des ersten Lichtleiters (122A, 222A, 622A, 722A) in Eingriff steht, sowie eine zweite Ferrule, die mit dem proximalen Leiterende (122P) des zweiten Lichtleiters in Eingriff steht; und
eine Aufnahmeanordnung (462, 562, 662, 762, 862, 962, 1062), aufweisend: (i) ein erstes Aufnahmeloch (468, 768, 868, 968, 1068), das dafür konfiguriert ist, die erste Ferrule (778, 1078) aufzunehmen, (ii) ein zweites Aufnahmeloch, das dafür konfiguriert ist, die zweite Ferrule aufzunehmen, (iii) einen Aufnahmeblock (464, 664, 764, 864, 964, 1064) und (iv) eine mit dem Aufnahmeblock (464, 664, 764, 864, 964, 1064) verbundene Trägerplatte (572, 772), wobei die Trägerplatte (572, 772) dafür konfiguriert ist, die erste Ferrule (778, 1078) und die zweite Ferrule in Eingriff zu bringen,
wobei das erste Aufnahmeloch (468, 768, 868, 968, 1068) und das zweite Aufnahmeloch jeweils im Aufnahmeblock (464, 664, 764, 864, 964, 1064) als V-Nuten ausgebildet sind.

2. Kathetersystem (100) nach Anspruch 1, wobei das Lichtleiterbündel (122, 152, 252, 352, 652) ferner aufweist: (i) eine erste Ferrulenanordnung (258, 358, 658, 758), die die erste Ferrule (778, 1078) und eine mit der ersten Ferrule (778, 1078) in Eingriff stehende erste Feder (776) aufweist, wobei die erste Feder (776) dafür konfiguriert ist, eine Einführkraft bereitzustellen, wenn die erste Ferrule (778, 1078) in das erste Aufnahmeloch (468, 768, 868, 968, 1068) eingeführt wird, und (ii) eine zweite Ferrulenanordnung, die die zweite Ferrule und eine mit der zweiten Ferrule in Eingriff stehende zweite Feder aufweist, wobei die zweite Feder dafür konfiguriert ist, eine Einführkraft bereitzustellen, wenn die zweite Ferrule in das zweite Aufnahmeloch eingeführt wird.

3. Kathetersystem (100) nach Anspruch 1 oder 2, wobei die erste Ferrule (778, 1078) und/oder die zweite Ferrule zumindest teilweise aus einem Keramikmaterial ausgebildet ist.

4. Kathetersystem (100) nach einem der Ansprüche 1 bis 3, wobei die erste Ferrule (778, 1078) und/oder die zweite Ferrule zumindest teilweise aus einem metallischen Material ausgebildet ist.

5. Kathetersystem (100) nach einem der Ansprüche 1 bis 4, wobei die Aufnahmeanordnung (462, 562, 662, 762, 862, 962, 1062) zumindest teilweise aus mindestens einem Material unter einem keramischen Material und einem metallischen Material ausgebildet ist.

6. Kathetersystem (100) nach einem der Ansprüche 1 bis 5, ferner aufweisend eine Ausrichtungsführung (470, 670, 770) und eine Ausrichtungsführungsaufnahme (260), wobei die Ausrichtungsführungsaufnahme (260) dafür konfiguriert ist, die Ausrichtungsführung (470, 670, 770) aufzunehmen und das Zusammenpassen der ersten Ferrule (778, 1078) mit dem ersten Aufnahmeloch (468, 768, 868, 968, 1068) und der zweiten Ferrule mit dem zweiten Aufnahmeloch zu verbessern.

7. Kathetersystem (100) nach einem der Ansprüche 1 bis 6, wobei jede der Ferrulen (766, 778, 1078) eine proximale Endfläche (778P) aufweist, und wobei die Trägerplatte (572, 772) dafür konfiguriert ist, mit jeder der proximalen Endflächen (778P) in Eingriff zu kommen.

8. Kathetersystem (100) nach einem der Ansprüche 1 bis 7, wobei die Trägerplatte (572, 772) (i) ein erstes Ausrichtungsloch aufweist, das dafür konfiguriert ist, einen ersten Führungsstrahl mit dem ersten Lichtleiter (122A, 222A, 622A, 722A) auszurichten, und (ii) ein zweites Ausrichtungsloch aufweist, das dafür konfiguriert ist, einen zweiten Führungsstrahl mit dem zweiten Lichtleiter auszurichten.

9. Kathetersystem (100) nach einem der Ansprüche 1 bis 8, wobei das erste Aufnahmeloch (468, 768, 868, 968, 1068) und das zweite Aufnahmeloch jeweils im Aufnahmeblock (464, 664, 764, 864, 964, 1064) ausgebildet sind, wobei das erste Aufnahmeloch (468, 768, 868, 968, 1068) und das zweite Aufnahmeloch auf einer gleichen gemeinsamen linearen Achse miteinander ausgerichtet sind.

10. Kathetersystem (100) nach einem der Ansprüche 1 bis 9, wobei die Aufnahmeanordnung (462, 562, 662, 762, 862, 962, 1062) ferner eine Halteanordnung (881, 1081) aufweist, die dafür konfiguriert ist, (i) die erste Ferrule (778, 1078) im ersten Aufnahmeloch (468, 768, 868, 968, 1068) und (ii) die zweite Ferrule im zweiten Aufnahmeloch zu halten.

11. Kathetersystem (100) nach Anspruch 10, wobei die Halteanordnung (881, 1081) eine Klemmleiste (882, 982, 1082) mit einem Kugel-Feder-Plunger aufweist, wobei der Kugel-Feder-Plunger dafür konfiguriert ist, (i) mit mindestens einem Abschnitt (114, 116) der ersten Ferrule (778, 1078) in Kontakt zu kommen, so dass die erste Ferrule (778, 1078) im ersten Aufnahmeloch (468, 768, 868, 968, 1068) gehalten wird, und (ii) mit mindestens einem Abschnitt (114, 116) der zweiten Ferrule in Kontakt zu kommen, so dass die zweite Ferrule im zweiten Aufnahmeloch gehalten wird.

12. Kathetersystem (100) nach Anspruch 11, wobei der Plunger (884, 984, 1084) in die Klemmleiste (882, 982, 1082) integriert ist.

13. Kathetersystem (100) nach Anspruch 11 oder 12, wobei die Klemmleiste (882, 982, 1082) derart konfiguriert ist, dass sie um eine Klemmleistenachse (882a) drehbar ist, wobei die Klemmleiste (882, 982, 1082) ein nachgiebiges Material aufweist, das dafür konfiguriert ist, eine Haltekraft über die Ferrulen (766, 778, 1078) zu verteilen, so dass jede Ferrule (766, 778, 1078) in einem entsprechenden Aufnahmeloch gehalten wird.

14. Kathetersystem (100) nach einem der Ansprüche 1 bis 13, wobei die Lichtquelle (124) einen Laser aufweist, und wobei sowohl der erste Lichtleiter (122A, 222A, 622A, 722A) als auch der zweite Lichtleiter eine optische Faser aufweist.

## Revendications

1. Système de cathéter (100) pour traiter un site de traitement (106) à l'intérieur ou à proximité d'une paroi de vaisseau (108A) ou d'une valve cardiaque, le système de cathéter (100) comprenant :
une source lumineuse (124) qui génère une énergie lumineuse ;
un premier guide de lumière (122A, 222A, 622A, 722A) qui reçoit l'énergie lumineuse provenant de la source lumineuse (124), le premier guide de lumière (122A, 222A, 622A, 722A) ayant une extrémité proximale de guide (122P) ;
un deuxième guide de lumière qui reçoit l'énergie lumineuse provenant de la source lumineuse (124), le deuxième guide de lumière ayant une extrémité proximale de guide (122P) ;
un faisceau de guides (122, 152, 252, 352, 652) qui est en communication optique avec la source lumineuse (124), le faisceau de guides (122, 152, 252, 352, 652) regroupant le premier guide de lumière (122A, 222A, 622A, 722A) et le deuxième guide de lumière, le faisceau de guides (122, 152, 252, 352, 652) comprenant une première virole (778, 1078) qui met en prise l'extrémité proximale de guide (122P) du premier guide de lumière (122A, 222A, 622A, 722A) et une deuxième virole qui met en prise l'extrémité proximale de guide (122P) du deuxième guide de lumière ; et
un ensemble réceptacle (462, 562, 662, 762, 862, 962, 1062) comprenant (i) un premier trou de réceptacle (468, 768, 868, 968, 1068) qui est configuré pour recevoir la première virole (778, 1078), (ii) un deuxième trou de réceptacle qui est configuré pour recevoir la deuxième virole, (iii) un bloc de réceptacle (464, 664, 764, 864, 964, 1064), et (iv) une plaque de support (572, 772) qui est couplée au bloc de réceptacle (464, 664, 764, 864, 964, 1064), la plaque de support (572, 772) étant configurée pour mettre en prise la première virole (778, 1078) et la deuxième virole ;
dans lequel le premier trou de réceptacle (468, 768, 868, 968, 1068) et le deuxième trou de réceptacle sont chacun formés dans le bloc de réceptacle (464, 664, 764, 864, 964, 1064) sous forme de rainures en V.

2. Système de cathéter (100) selon la revendication 1, dans lequel le faisceau de guides (122, 152, 252, 352, 652) comprend en outre (i) un premier ensemble de virole (258, 358, 658, 758) comprenant la première virole (778, 1078) et un premier ressort (776) qui met en prise la première virole (778, 1078), le premier ressort (776) étant configuré pour fournir une force d'insertion lorsque la première virole (778, 1078) est insérée dans le premier trou de réceptacle (468, 768, 868, 968, 1068), et (ii) un deuxième ensemble de virole comprenant la deuxième virole et un deuxième ressort qui met en prise la deuxième virole, le deuxième ressort étant configuré pour fournir une force d'insertion lorsque la deuxième virole est insérée dans le deuxième trou de réceptacle.

3. Système de cathéter (100) selon l'une quelconque des revendications 1 ou 2, dans lequel au moins l'une de la première virole (778, 1078) et de la deuxième virole est formée au moins partiellement à partir d'un matériau en céramique.

4. Système de cathéter (100) selon l'une quelconque des revendications 1 à 3, dans lequel au moins l'une de la première virole (778, 1078) et de la deuxième virole est formée au moins partiellement à partir d'un matériau métallique.

5. Système de cathéter (100) selon l'une quelconque des revendications 1 à 4, dans lequel l'ensemble réceptacle (462, 562, 662, 762, 862, 962, 1062) est formé au moins partiellement à partir d'au moins l'un parmi un matériau en céramique et un matériau métallique.

6. Système de cathéter (100) selon l'une quelconque des revendications 1 à 5, comprenant en outre un guide d'alignement (470, 670, 770) et un récepteur de guide d'alignement (260), le récepteur de guide d'alignement (260) étant configuré pour recevoir le guide d'alignement (470, 670, 770) et pour améliorer l'accouplement de la première virole (778, 1078) avec le premier trou de réceptacle (468, 768, 868, 968, 1068) et de la deuxième virole avec le deuxième trou de réceptacle.

7. Système de cathéter (100) selon l'une quelconque des revendications 1 à 6, dans lequel chacune des viroles (766, 778, 1078) comprend une face d'extrémité proximale (778P), et dans lequel la plaque de support (572, 772) est configurée pour mettre en prise chacune des faces d'extrémité proximale (778P).

8. Système de cathéter (100) selon l'une quelconque des revendications 1 à 7, dans lequel la plaque de support (572, 772) comprend (i) un premier trou d'alignement qui est configuré pour aligner un premier faisceau de guidage avec le premier guide de lumière (122A, 222A, 622A, 722A), et (ii) un deuxième trou d'alignement qui est configuré pour aligner un deuxième faisceau de guidage avec le deuxième guide de lumière.

9. Système de cathéter (100) selon l'une quelconque des revendications 1 à 8, dans lequel le premier trou de réceptacle (468, 768, 868, 968, 1068) et le deuxième trou de réceptacle sont chacun formés dans le bloc de réceptacle (464, 664, 764, 864, 964, 1064), le premier trou de réceptacle (468, 768, 868, 968, 1068) et le deuxième trou de réceptacle étant alignés sur un même axe linéaire l'un par rapport à l'autre.

10. Système de cathéter (100) selon l'une quelconque des revendications 1 à 9, dans lequel l'ensemble réceptacle (462, 562, 662, 762, 862, 962, 1062) comprend en outre un ensemble de retenue (881, 1081) qui est configuré pour retenir (i) la première virole (778, 1078) dans le premier trou de réceptacle (468, 768, 868, 968, 1068), et (ii) la deuxième virole dans le deuxième trou de réceptacle.

11. Système de cathéter (100) selon la revendication 10, dans lequel l'ensemble de retenue (881, 1081) comprend une barre de serrage (882, 982, 1082) ayant un poussoir à bille et ressort, le poussoir à bille et ressort étant configuré pour entrer en contact (i) avec au moins une partie (114, 116) de la première virole (778, 1078) de sorte que la première virole (778, 1078) soit retenue dans le premier trou de réceptacle (468, 768, 868, 968, 1068), et (ii) avec au moins une partie (114, 116) de la deuxième virole de sorte que la deuxième virole soit retenue dans le deuxième trou de réceptacle.

12. Système de cathéter (100) selon la revendication 11, dans lequel le poussoir (884, 984, 1084) est intégré dans la barre de serrage (882, 982, 1082).

13. Système de cathéter (100) selon l'une quelconque des revendications 11 à 12, dans lequel la barre de serrage (882, 982, 1082) est conçue pour pouvoir tourner autour d'un axe de barre de serrage (882a), la barre de serrage (882, 982, 1082) comprenant un matériau souple qui est configuré pour répartir une force de retenue sur les viroles (766, 778, 1078) de sorte que chaque virole (766, 778, 1078) soit retenue à l'intérieur d'un trou de réceptacle correspondant.

14. Système de cathéter (100) selon l'une quelconque des revendications 1 à 13, dans lequel la source lumineuse (124) comprend un laser, et dans lequel chacun du premier guide de lumière (122A, 222A, 622A, 722A) et du deuxième guide de lumière comprend une fibre optique.
